# EUROPÄISCHE PATENTSCHRIFT

(11) **EP 4 213 734 B1**
(45) Veröffentlichungstag und Bekanntmachung des Hinweises auf die Patenterteilung: **24.04.2024**
(21) Anmeldenummer: 20780146.5
(22) Anmeldetag: 23.09.2020
(51) Int. Cl.: A61B 6/00, H01J 35/30, A61B 6/40, A61B 6/42

(54) **COMPUTERTOMOGRAPH UND VERFAHREN ZUM BETRIEB EINES COMPUTERTOMOGRAPHEN**
COMPUTER TOMOGRAPHY MACHINE AND METHOD FOR OPERATING A COMPUTER TOMOGRAPHY MACHINE
MACHINE DE TOMOGRAPHIE ASSISTÉE PAR ORDINATEUR ET PROCÉDÉ DE FONCTIONNEMENT D'UNE MACHINE DE TOMOGRAPHIE ASSISTÉE PAR ORDINATEUR

(30) Priorität: 19.09.2020 DE 102020124474
(43) Veröffentlichungstag der Anmeldung: 26.07.2023
(73) Patentinhaber: Esspen GmbH, 91052 Erlangen (DE)
(72) Erfinder: MOHAMMADI, Zahra, 91052 Erlangen (DE)
(74) Vertreter: Meyer, Rudolf
(86) Internationale Anmeldenummer: PCT/EP2020/076474
(87) Internationale Veröffentlichungsnummer: WO 2022/058036

(56) Entgegenhaltungen:
- EP-A1- 3 569 148
- WO-A1-2009/115982
- DE-A1-102010 020 604
- DE-A1-102016 013 533

## Beschreibung

Die Erfindung betrifft einen Computertomographen, welcher einen statischen Strahler-Detektor-Ring, das heißt einen während des Betriebs des Computertomographen nicht rotierenden Strahler-Detektor-Ring, umfasst. Ferner betrifft die Erfindung ein Verfahren zum Betrieb eines solchen Computertomographen.

Computertomographen können grundsätzlich entweder mit rotierenden Strahler-Detektor-Einheiten oder mit ortsfest angeordneten Röntgenstrahlern und zugehörigen Detektoren arbeiten. Eine mögliche Bauform eines Computertomographen, welcher eine nicht rotierende Strahler-Detektor-Einheit aufweist, ist in der WO 2018/086744 A2 beschrieben. Die Strahler-Detektor-Einheit ist aus mehreren Strahler-Detektor-Elementen aufgebaut ist, wobei jedes Strahler-Detektor-Element eine zur Emission von Röntgenstrahlung vorgesehene Anodenanordnung sowie einen zur Detektion von Röntgenstrahlung vorgesehenen Detektor aufweist, und jede Anodenanordnung ist Teil einer mehrere Elektronen-Emitter umfassenden Strahler-Anordnung.

Ein weiterer Computertomograph, welcher nicht rotierende Röntgenquellen sowie zugehörige Detektoren aufweist, ist zum Beispiel aus der WO 2009/115982 A1 bekannt. Dieser Computertomograph soll insbesondere für Anwendungen in der Kardiologie geeignet sein.

Die DE 10 2010 020 604 A1 beschreibt eine Bildaufnahmevorrichtung, welche eine ringförmige Gantry mit einer in ihr rotierenden Rotoranordnung mit einer Strahlungsquelle sowie mindestens einem Strahlungsdetektor umfasst. Die Gantry weist ein zum seitlichen Öffnen der Gantry herausnehmbares Gantrysegment auf. Das herausnehmbare Gantrysegment ergänzt eine Rotoranordnung, welche sich über einen Winkel von ca. 270° erstreckt, zu einer geschlossenen Ringform. Insgesamt ist die Gantry an einer Tragstruktur im Raum bewegbar angeordnet.

Die EP 1474 040 B1 offenbart eine röntgentechnische Bildgebungsvorrichtung mit einem Gantry-Rahmen, in welchem eine Röntgenstrahlungsquelle und ein zugehöriger Detektor rotierbar gelagert sind. Von der insgesamt ringförmigen Gantry ist ein Segment abnehmbar, um ein abzubildendes Objekt im zentralen Bildgebungsbereich des Gantry-Rings positionieren zu können. Führungsvorrichtungen für die Strahlungsquelle und für den Detektor sind auch im abnehmbaren Segment zu finden.

Ein weiterer röntgentechnischer Apparat, welcher eine insgesamt ringförmige Strahler-Detektor-Anordnung mit einem zum Zwecke des Öffnens des Rings beweglichen Segment aufweist, ist in der US 6,113,264 A beschrieben. Das bewegliche Segment ist hierbei in Umfangsrichtung der ringförmigen Strahler-Detektor-Anordnung verschiebbar.

Die Dokumente US 7,001,045 B2 und EP 3 646 793 A2 beschreiben verschiedene Bauformen von Computertomographen mit ringförmigen Strahler-Detektor-Baugruppen, die in vielfältiger Weise verstellbar sind.

Computertomographen, welche als Ganzes verfahrbar sind, sind zum Beispiel in den Dokumenten WO 2010/078481 A1 und DE 10 2016 208 123 B4 beschrieben.

Die DE 10 2010 028 438 A1 beschreibt ein als C-Arm-Gerät ausgebildetes röntgentechnisches Gerät, welches Kohlenstoffnanoröhren als Elektronenemitter aufweist. Mit Hilfe mehrerer räumlich unterschiedlich angeordneter Röntgenstrahlungsquellen sind Abtasttrajektorien realisierbar, welche in der DE 10 2010 028 438 A1 im Detail beschrieben sind.

Die EP 3 569 148 A1 hat ein Verfahren zur Aufnahme von einem Bilddatensatz mit einem Röntgendetektor zum Gegenstand. Im Rahmen dieses Verfahrens ist ein erstes Erfassen von einer ersten Aufnahme mit einer ersten Position des Brennflecks vorgesehen. Anschließend wird der Brennfleck von der ersten Position zu einer zweiten Position verschoben. In dieser Einstellung wird eine zweite Aufnahme erfasst. Schließlich werden nach der EP 3 569 148 A1 beide Aufnahmen zu einem Bilddatensatz kombiniert.

Der Erfindung liegt die Aufgabe zugrunde, gegenüber dem Stand der Technik weiterentwickelte Möglichkeiten der röntgentechnischen Bildgebung mit einem statischen Strahler-Detektor-Ring anzugeben, wobei eine gute Handhabbarkeit und hervorragende Qualität der Bildgebung bei nicht zu kompliziertem apparativen Aufbau gegeben sein soll.

Diese Aufgabe wird erfindungsgemäß gelöst durch einen Computertomographen mit den Merkmalen des Anspruchs 1. Ebenso wird die Aufgabe gelöst durch ein Verfahren zum Betrieb eines Computertomographen gemäß Anspruch 11. Im Folgenden im Zusammenhang mit dem Betriebsverfahren erläuterte Ausgestaltungen und Vorteile der Erfindung gelten sinngemäß auch für die Vorrichtung, das heißt den Computertomographen, und umgekehrt.

Der Computertomograph umfasst einen statischen Strahler-Detektor-Ring, welcher aus einer ungeraden Anzahl n an Strahler-Detektor-Elementen aufgebaut ist, von denen ein einziges unter Öffnung des Strahler-Detektor-Rings gegenüber den übrigen, zusammen eine C-Form beschreibenden Strahler-Detektor-Elementen verschiebbar ist, wobei jedes Strahler-Detektor-Element eine zur Emission von Röntgenstrahlung vorgesehene Anodenanordnung, welche sich über einen Winkel α von mindestens 0,9 × 360°/n am Umfang des Strahler-Detektor-Rings erstreckt, sowie einen zur Detektion von Röntgenstrahlung vorgesehenen Detektor, der sich innerhalb desselben Strahler-Detektor-Elements über einen Winkel β von mindestens 0,95 × 360°/n erstreckt, aufweist, und wobei jede Anodenanordnung Teil einer mehrere Elektronen-Emitter umfassenden Strahler-Anordnung ist, in welcher jeder Elektronen-Emitter in Zusammenwirkung mit einer Elektrodenanordnung zur Erzeugung eines Brennflecks an einer von mindestens drei wählbaren Positionen auf der Anodenanordnung ausgebildet ist. Die wahlweise einstellbaren Positionen des mit ein und demselben Elektronen-Emitter erzeugbaren Brennflecks sind hierbei insbesondere in Umfangsrichtung des Strahler-Detektor-Rings nebeneinander, das heißt in unterschiedlichen Winkelpositionen um die Mittelachse des Computertomographen, angeordnet, insbesondere äquidistant angeordnet. Die Umschaltung zwischen verschiedenen Brennfleckpositionen, indem die Einstellung einer Elektrodenanordnung stufenweise geändert wird, wird als Beam Toggling bezeichnet.

Das anmeldungsgemäße Betriebsverfahren geht davon aus, dass ein Computertomograph genutzt wird, der einen nicht rotierenden Strahler-Detektor-Ring umfasst, der aus einer ungeraden Anzahl an Strahler-Detektor-Elementen, das heißt Segmenten, aufgebaut ist, von welchen ein einziges Segment zum Öffnen des Strahler-Detektor-Rings ausgebildet ist, wobei sowohl in den festen Strahler-Detektor-Elementen als auch im zu öffnenden Strahler-Detektor-Element eine Mehrzahl an Elektronen-Emittern angeordnet ist, die jeweils dazu ausgebildet sind, mit Hilfe von Elektronenstrahlen beeinflussenden Elektroden einen Brennfleck mit variabler Position auf einer dem Strahler-Detektor-Element zugehörigen Anode zu erzeugen, so dass die Gesamtzahl möglicher Brennfleckpositionen einem Mehrfachen der Anzahl an Elektronen-Emittern entspricht, und wobei der maximale Winkelabstand zwischen zwei in Umfangsrichtung des Strahler-Detektor-Rings innerhalb ein und desselben Strahler-Detektor-Elementes nebeneinander angeordneten Brennfleckpositionen geringer ist als der minimale Winkelabstand zwischen Brennfleckpositionen zweier benachbarter Strahler-Detektor-Elemente. Das Betriebsverfahren umfasst folgende Schritte:
- Positionierung des Strahler-Detektor-Rings um ein Untersuchungsobjekt, wobei der Strahler-Detektor-Ring zunächst geöffnet ist und spätestens in einer zur Durchführung einer röntgentechnischen Untersuchung vorgesehenen Position geschlossen wird,
- Richten eines fächerförmigen Röntgenstrahlenbündels, welches von einem ersten Brennfleck ausgeht, auf das Untersuchungsobjekt, wobei Röntgenstrahlung von Detektoren mindestens zweier Strahler-Detektor-Elemente detektiert wird,
- Erzeugen eines zweiten Brennflecks, welcher am Umfang des Strahler-Detektor-Rings gegenüber dem ersten Brennfleck um einen ersten Differenzwinkel versetzt und nicht notwendigerweise direkt neben dem ersten Brennfleck angeordnet ist,
- Erzeugen weiterer Brennflecke, welche am Umfang des Strahler-Detektor-Rings jeweils gegenüber dem vorherigen Brennfleck um einen Differenzwinkel versetzt sind, wobei der Differenzbetrag zwischen zwei aufeinanderfolgenden Differenzwinkeln geringer als die Differenz zwischen dem minimalen Winkelabstand zwischen Brennfleckpositionen in benachbarten Strahler-Detektor-Elementen und dem maximalen Winkelabstand zwischen zwei benachbarten Brennfleckpositionen innerhalb ein und desselben Strahler-Detektor-Elementes ist.

Die Erfindung geht von der Überlegung aus, dass ein Computertomograph, dessen Strahler-Detektor-Ring geöffnet werden kann, gegenüber herkömmlichen Computertomographen mit permanent geschlossenem Strahler-Detektor-Ring Vorteile bei der Vorbereitung röntgentechnischer Aufnahmen bietet. Beispielsweise kann der zu öffnende Strahler-Detektor-Ring im offenen Zustand von der Seite über einen Tisch, auf welchem der Patient liegt, geschoben werden. Trotz der Möglichkeit, den Strahler-Detektor-Ring zu öffnen, ist ein massesparender Aufbau bei einer gleichzeitig hohen Anzahl an Brennfleckpositionen dadurch begünstigt, dass pro Elektronen-Emitter verschiedene Brennfleckpositionen wahlweise einstellbar sind. Insgesamt nehmen die Anoden, welche am Umfang der Strahler-Anordnung positioniert sind, mindestens 90 % des Umfangs, das heißt 324°, ein. Aufgrund der ungeraden Anzahl an Strahler-Detektor-Elementen liegt niemals eine Stoßstelle zwischen zwei Strahler-Detektor-Elementen einer weiteren derartigen Stoßstelle exakt diametral gegenüber. Befindet sich ein Brennfleck im Randbereich eines Strahler-Detektor-Elements, das heißt nahe einer Stoßstelle, so trifft von diesem Brennfleck ausgehende Röntgenstrahlung auf zwei in Umfangsrichtung nebeneinander angeordnete Röntgendetektoren, bei welchen es sich beispielsweise um photonenzählende Detektoren handelt. Insbesondere kommt die Verwendung von Zeilendetektoren in Betracht. In diesem Zusammenhang wird auf die WO 2019/057339 A1 hingewiesen. Generell sind auch Szintillationsdetektoren, welche beliebig geformt sein können, als Röntgendetektoren verwendbar. Im Vergleich zu den Anoden des Strahler-Detektor-Rings erstrecken sich die Detektoren in jedem Fall über einen noch größeren Winkel, nämlich über mindestens 95 % des Umfangs, das heißt über einen auf die einzelnen Segmente des Strahler-Detektor-Rings aufgeteilten Winkel von insgesamt mindestens 342°.

Im Vergleich zum minimalen Winkelabstand, welcher zwischen Brennfleckpositionen in benachbarten Strahler-Detektor-Elementen gegeben sein kann, sind die möglichen Brennfleckpositionen innerhalb ein und desselben Strahler-Detektor-Elementes wesentlich enger gestaffelt. Würde man sämtliche möglichen Brennfleckpositionen in Umfangsrichtung des Strahler-Detektor-Rings nacheinander auswählen, so würde auf eine Anzahl kleiner Winkelabstände regelmäßig, nämlich beim Wechsel von einem Strahler-Detektor-Element zum nächsten Strahler-Detektor-Element, ein weitaus größerer Sprung in der Winkeleinstellung erfolgen. Eine Vergleichmäßigung der Winkelabstände zwischen einer Brennfleckposition und der im Zeitablauf nächsten Brennfleckposition ist durch das anmeldungsgemäße Betriebsverfahren gegeben. Hierbei können durch die sukzessive Umschaltung zwischen verschiedenen Brennfleckpositionen mehrere Umläufe um die Mittelachse des Strahler-Detektor-Rings beschrieben werden, wobei erst nach einer Mehrzahl an Umläufen sämtliche mögliche Brennfleckpositionen eingenommen worden sind. Insbesondere werden bei jedem einzelnen Umlauf Brennflecke in voneinander abweichenden Einstellungen der die Elektronenstrahlen beeinflussenden Elektroden erzeugt. Dies kann beispielsweise bedeuten, dass während eines virtuellen Röntgenquellen-Umlaufs eine erste Elektrodenanordnung derart eingestellt wird, dass ein Elektronenstrahl in einer mittleren, neutralen Ausrichtung verbleibt, wogegen eine zweite, zu einem anderen Elektronen-Emitter gehörende Elektrodenanordnung dafür sorgt, dass der entsprechende Elektronenstrahl im Vergleich zu seiner neutralen Ausrichtung in einer bestimmten Richtung ausgelenkt wird, was mit einem Versatz des Brennpunktes am Umfang des Strahler-Detektor-Rings einhergeht.

Möglichkeiten der sukzessiven Verstellung der Winkeleinstellung der Röntgenstrahlung, das heißt der Umschaltung zwischen verschiedenen möglichen Brennfleckpositionen, während des Betriebs des Computertomographen werden im Folgenden anhand eines vereinfachten Zahlenbeispiels erläutert:
Ein Strahler-Detektor-Ring sei aus lediglich drei Strahler-Detektor-Elementen aufgebaut, welche sich jeweils über einen Winkel von annähernd 120° erstrecken. Eine Stoßstelle zwischen dem ersten und dem dritten Strahler-Detektor-Element befinde sich in der 0°-Position. Die Mitten der drei Segmente - in Umfangsrichtung des Strahler-Detektor-Rings betrachtet - befinden sich somit bei 60°, 180° und 300°.

Der Einfachheit halber wird angenommen, dass jedes Strahler-Detektor-Element lediglich zwei Elektronenemitter aufweist, wobei der von einem Emitter ausgehende Elektronenstrahl auf drei verschiedene Arten auf die zugehörige Anodenanordnung gerichtet werden kann:
Der Elektronenstrahl ist entweder mit einer mittleren Ausrichtung oder mit Ablenkung im Uhrzeigersinn oder mit Ablenkung im Gegenuhrzeigersinn auf die Anode gerichtet, wobei sich die Richtungsangaben der Ablenkung auf die Umfangsrichtung des Strahler-Detektor-Rings beziehen.

Gleichmäßige Abstände zwischen den möglichen Brennfleckpositionen innerhalb des ersten Strahler-Detektor-Elements sind beispielsweise mit folgenden Brennfleckpositionen gegeben: 20°, 36°, 52°, 68°, 84°, 100°. Hierbei sind die ersten drei Werte dem ersten Elektronemitter und die Werte 68°, 84°, 100° dem zweiten Elektronenemitter zugeordnet. Die mittleren Ausrichtungen der Elektronenstrahlen entsprechen den Brennfleckpositionen 36° (erster Elektronenemitter) beziehungsweise 84° (zweiter Elektronenemitter). In dem diskutierten Zahlenbeispiel, welches von einer sehr niedrigen Anzahl an Elektronenemittern ausgeht, seien die Elektronenstrahlen, verglichen mit der jeweils mittleren Ausrichtung, derart ablenkbar, dass sich Brennfleckpositionen ergeben, die um ± 16° gegenüber derjenigen Brennfleckposition versetzt sind, die sich bei der mittleren Ausrichtung des Elektronenstrahls ergibt. Zwischen einer innerhalb des ersten Strahler-Detektor-Elements liegenden Brennfleckposition und der nächsten möglichen Brennfleckposition innerhalb desselben Strahler-Detektor-Elements sind damit stets Winkelabstände von 16° gegeben.

In analoger Weise sind innerhalb des zweiten Strahler-Detektor-Elements Brennfleckpositionen bei 140°, 156°, 172°, 188°, 204°, 220° und innerhalb des dritten Strahler-Detektor-Elements Brennfleckpositionen bei 260°, 276°, 292°, 308°, 324°, 340° wählbar. Das erste Strahler-Detektor-Element stellt das zu öffnende Segment des Strahler-Detektor-Rings dar. Das zweite Strahler-Detektor-Element ist mit dem dritten Strahler-Detektor-Element fest verbunden. Ungeachtet der Tatsache, dass sich die Strahler-Detektor-Elemente hinsichtlich ihrer mechanischen Funktion voneinander unterscheiden, sind alle Strahler-Detektor-Elemente, was ihre röntgentechnische Funktion, insbesondere die Anordnung der Röntgenquellen, das heißt Brennflecke auf den Anoden, sowie der Röntgendetektoren, betrifft, gleich aufgebaut. Bei der Durchführung und Auswertung einer computertomographischen, das heiß röntgentechnischen Untersuchung braucht daher keine Rücksicht darauf genommen werden, inwieweit ein Strahler-Detektor-Element gegenüber den übrigen Strahler-Detektor-Elementen verstellbar ist.

Um die gewünschte Verstellbarkeit eines der Strahler-Detektor-Elemente sicherzustellen und zugleich sämtliche Strahler-Detektor-Elemente, was ihre röntgentechnische Funktion betrifft, gleichartig zu gestalten, kann der zur Verfügung stehende 120°-Bauraum innerhalb eines jeden Strahler-Detektor-Elementes nur zum Teil durch die jeweilige Anodenanordnung ausgenutzt werden. Zwischen der äußersten möglichen Brennfleckposition eines Strahler-Detektor-Elementes und der hiervon am geringsten entfernten Brennfleckposition des nächsten Strahler-Detektor-Elementes ist daher ein Winkelabstand gegeben, welcher wesentlich größer ist als die Winkelabstände zwischen den Brennfleckpositionen innerhalb ein und desselben Strahler-Detektor-Elements. Im vorliegenden Fall sind folgende Elementübergreifende minimale Winkelabstände zwischen Brennfleckpositionen verschiedener Strahler-Detektor-Elemente gegeben: Zwischen dem ersten und dem dritten Element (20° bzw. 340°) ein Winkelabstand von 40° und zwischen dem ersten und dem zweiten Element (100° bzw. 140°) sowie zwischen dem zweiten und dem dritten Element (220° bzw. 260°) jeweils ebenfalls ein Winkelabstand von 40°.

Würde man nun im Uhrzeigersinn (oder auch im Gegenuhrzeigersinn) sämtliche möglichen Brennfleckpositionen nacheinander einstellen, was eine mechanische Rotation einer Röntgenquelle um die Längsachse des Computertomographen simuliert, so würde auf fünf Winkeländerungen um jeweils 16° stets eine Winkeländerung von 40° folgen, an welche sich wiederum fünf Sprünge um 16° anschließen.

Dieser Ungleichmäßigkeit der Winkelsprünge kann abgeholfen werden, indem ein neuartiges Schema angewandt wird, nach welchem nacheinander verschiedene Brennfleckpositionen eingestellt werden:
Beginnend mit der im Uhrzeigersinn ersten möglichen Position eines Brennflecks, das heißt der 20°-Position, werden sechs virtuelle Umläufe gestartet, wobei in dem vereinfachten Beispiel in jedem Umlauf lediglich drei Brennfleckpositionen nacheinander gewählt werden, die im Durchschnitt um 120° gegeneinander versetzt sind. Im Einzelnen lautet eine mögliche Reihenfolge an Brennfleckpositionen: 20°, 140°, 276°, 52°, 172°, 308°, 84°, 204°, 340°, 100°, 220°, 324°, 68°, 188°, 292°, 36°, 156°, 260°, womit nach den sechs virtuellen Umläufen jede der insgesamt 18 möglichen Brennfleckpositionen genau einmal gewählt wurde. Von den durchschnittlichen 120°-Winkelabständen wird hierbei um nicht mehr als 16° nach oben oder unten abgewichen, wobei acht Mal der Winkelabstand von 120° und je fünf Mal ein Winkelabstand von 136° beziehungsweise 104° gegeben ist und niemals der Wert 104° auf den Wert 136° (oder umgekehrt) folgt. Dies gilt auch für einen erneuten Start der sechs virtuellen Umläufe, das heißt für den Wechsel von der 260°-Position auf die 20°-Position, was einem Differenzwinkel von genau 120° entspricht.

Als Differenzwinkel tauchen in dem betrachteten, vereinfachten Beispiel somit lediglich drei verschiedene Werte, nämlich die Werte 104°, 120°, 136°, auf, wobei der Differenzbetrag zwischen zwei aufeinander folgenden Differenzwinkeln, das heißt der Betrag von 0° oder 16°, geringer als der Winkelabstand zwischen den am nächsten beieinander liegenden Brennflecken zweier Strahler-Detektor-Elemente ist. Im vorliegenden Fall ist der Differenzbetrag sogar geringer als die Hälfte des genannten, sich über eine Segmentgrenze erstreckenden Winkelabstandes von 40°. Zieht man von diesem segmentübergreifenden Winkelabstand den im erläuterten Beispiel einheitlichen Winkelabstand innerhalb der Segmente in Höhe von 16° ab, so ergibt sich eine Differenz von 24°, welche immer noch größer als der größte auftretende Differenzbetrag zwischen zeitlich aufeinander folgenden Differenzwinkeln ist. Trotz der aufgrund der Segmentierung des Strahler-Detektor-Rings und dessen Öffnungsmechanismus gegebenen ungleichmäßigen Verteilung der Brennflecke am Umfang des Strahler-Detektor-Rings ist damit eine in vergleichsweise gleichmäßigen Winkelsprüngen, die eine Rotation einer Röntgenquelle simulieren, ablaufende Durchleuchtung eines Untersuchungsobjektes mit Röntgenstrahlung in Form jeweils von einem Brennfleck ausgehender fächerförmiger Strahlenbündel möglich.

Jeder Brennfleck befindet sich auf einer Anodenanordnung, die entweder eine einzige oder mehrere flüssigkeitsgekühlte oder ungekühlte Anoden pro Röntgenröhre umfassen kann. Beispielsweise kommen Anoden zum Einsatz, wie sie in der DE 10 2017 008 810 A1 beschrieben sind. Unabhängig von der Gestaltung der Anoden kann der Computertomograph dazu ausgebildet sein, dass durch die Strahler-Detektor-Elemente gleichzeitig zwei oder mehr am Umfang des Strahler-Detektor-Rings gegeneinander versetzte Brennflecke erzeugt werden. Im Fall von genau zwei Brennflecken und dementsprechend zwei Röntgenstrahlenbündeln, mit denen ein interessierendes Volumen untersucht wird, spricht man auch von einer Stereo Taktik der röntgentechnischen Bildgebung.

Was die Gestaltung der Kathoden, welche als Elektronen-Emitter des Computertomographen zum Einsatz kommen, betrifft, können zum Beispiel Möglichkeiten gewählt werden, die in der WO 2019/057338 A1, welche die Priorität der genannten Patentanmeldung der DE 10 2017 008 810 A1 in Anspruch nimmt, gewählt werden.

Möglichkeiten der Ansteuerung, welche auch im vorliegenden Fall genutzt werden können, sind zum Beispiel in der WO 2019/042587 A2 beschrieben. Bei der Herstellung der zur Emission von Elektronen ausgebildeten Kathoden kann zum Beispiel auf jegliche Lösungen zurückgegriffen werden, die in den Dokumenten WO 2018/086737 A1 und WO 2018/141485 A1 erwähnt sind.

Insgesamt können die Röntgenröhren des Computertomographen dazu ausgebildet sein, eine Folge von Röntgenpulsen zu erzeugen, welche sich hinsichtlich verschiedenster Parameter, unter anderem Dauer, Intensität und Röntgendosis der einzelnen Pulse sowie Frequenz der Röntgenstrahlung, voneinander unterscheiden. Zu diesem Zweck kann unter anderem eine Variation der an einer Anode anliegenden Spannung sowie eine Variation der von den Kathoden ausgehenden Elektronenströme vorgesehen sein. Diese die Röntgenquellen betreffenden Variationsmöglichkeiten spielen ihre Vorteile besonders in der Kombination mit Photonen zählenden Detektoren, welche zur Unterscheidung verschiedener Röntgenfrequenzen geeignet sind, das heißt für Multi-Energy- und Multi-Dosis-Betriebsmodi prädestiniert sind, aus.

Bei dem Computertomographen kann es sich um ein mobiles oder um ein stationäres röntgentechnisches Gerät handeln. Der Computertomograph ist unter anderem für Untersuchungen im Thoraxbereich geeignet.

Nachfolgend wird ein Ausführungsbeispiel der Erfindung anhand einer Zeichnung näher erläutert. Hierin zeigen:
- Fig. 1: einen Computertomographen in einer Übersichtsdarstellung,
- Fig. 2: in einer Darstellung analog Figur 1 den Computertomographen mit geöffnetem Strahler-Detektor-Ring,
- Fig. 3 und 4: in verschiedenen Vergrößerungen Details des Computertomographen,
- Fig. 5 bis 7: eine Röntgenröhre des Computertomographen,
- Fig. 8: ein aus Röntgenröhre und zugehörigem Detektor aufgebautes Strahler-Detektor-Element des Computertomographen,
- Fig. 9 und 10: in schematisierten Darstellungen Einzelheiten des Computertomographen.

Ein insgesamt mit dem Bezugszeichen 1 gekennzeichneter Computertomograph umfasst eine feststehende Gantry 2, wobei der Begriff "feststehend" dahingehend zu verstehen ist, dass bei der Gewinnung röntgentechnischer Aufnahmen keine Rotation einer Strahler-Detektor-Einheit um die Mittelachse MA der Gantry 2 gegeben ist. Vielmehr sind mit Hilfe um den gesamten Umfang der Gantry 2 verteilter Röntgenröhren 3 und zugehöriger Röntgendetektoren 4 fächerförmige Bündel an Röntgenstrahlung RS erzeugbar, welche jeweils von einem Brennfleck BF auf einer Anode 6 der Röntgenröhre 3 ausgehen. Den einzelnen Röntgenröhren 3 sind jeweils mehrere Kathoden 5, 25 als Elektronenemitter zuzurechnen. Im skizzierten Ausführungsbeispiel weist jede Röntgenröhre 3 ein einzige langgestreckte Anode 6 auf, die in diesem Fall zugleich als Anodenanordnung 9 gilt. Alternativ kann die Anodenanordnung 9 einer Röntgenröhre 3 aus einer Mehrzahl an Anoden 6 aufgebaut sein.

Die Gantry 2 ist an einem fahrbaren Gestell 7 in vielfach verstellbarer Weise angebracht. Unter anderem ist ein Kippen der Gantry 2 um eine waagerechte, die Mittelachse MA orthogonal schneidende Kippachse möglich. Ebenso kann die Gantry 2 in Längsrichtung der horizontal angeordneten Mittelachse MA verschoben werden. Darüber hinaus sind auch beschränkte Verstellungen der Gantry 2 in deren Umfangsrichtung möglich. Auch die Möglichkeit eines Anhebens oder Absenkens der gesamten Gantry 2 ist gegeben. Im Ausführungsbeispiel ist zusätzlich zum fahrbaren Gestell 7 eine gesonderte Bedien- und Auswerteeinheit 8 vorhanden. Eine bauliche Zusammenfassung von fahrbarem Gestell 7 und Bedien- und Auswerteeinheit 8 ist ebenfalls denkbar.

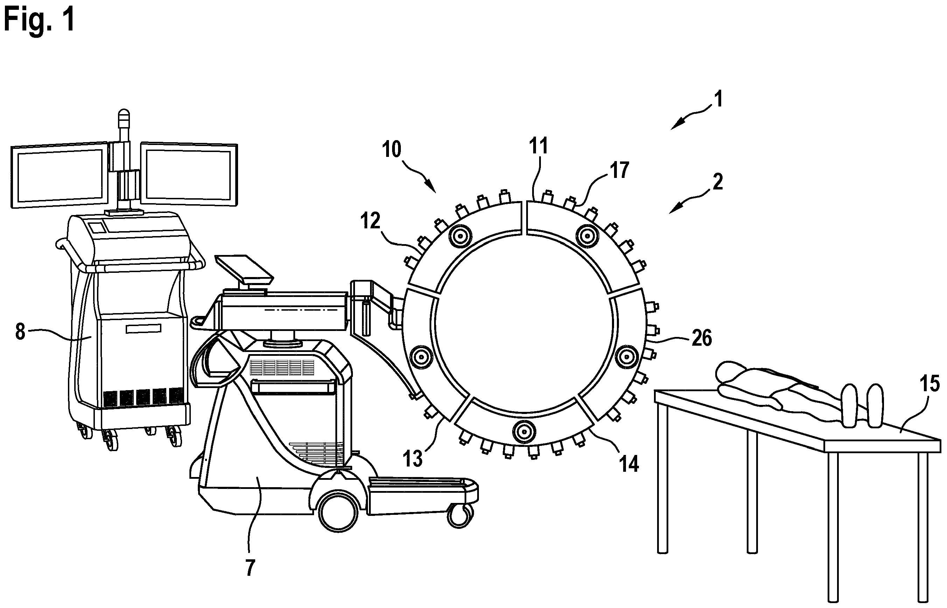

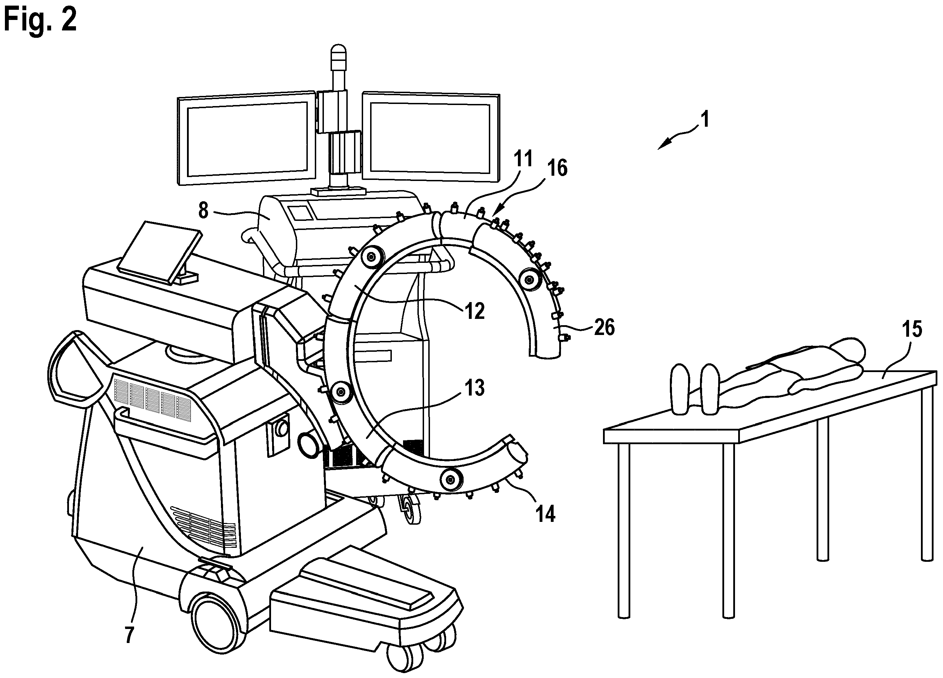

Ein die Gantry 2 umschließendes Gehäuse ist nicht vorgesehen. Vielmehr ist die gesamte Gantry 2 als Strahler-Detektor-Ring 10 aufgebaut, der aus insgesamt fünf Strahler-Detektor-Elementen 11, 12, 13, 14, 26 gebildet ist. Hierbei stellen die Strahlen-Detektor-Elemente 11, 12, 13, 14 feststehende, starr miteinander verbundene Elemente dar, wogegen das Strahler-Element 26 verschiebbar ist, um den Strahler-Detektor-Ring 10 zu öffnen. Das Öffnen erfolgt, um mit dem Strahler-Detektor-Ring 10 eine Patientenliege 15 zu umschließen. Der Öffnungsmechanismus ist als Schiebemechanismus 16 aufgebaut. Ausgehend vom geschlossenen Strahler-Detektor-Ring 10, wie in Figur 1 dargestellt, wird das Strahler-Detektor-Element 26 zunächst etwas in Längsrichtung der Mittelachse MA verschoben, das heißt aus dem Strahler-Detektor-Ring 10 herausgehoben. In diesem Zustand, in welchem das Strahler-Detektor-Element 26 neben der C-förmigen Anordnung der übrigen Strahler-Detektor-Elemente 11, 12, 13, 14 positioniert ist, kann das Strahler-Detektor-Element 26 in Umfangsrichtung der C-förmigen Anordnung 11, 12, 13, 14 verschoben werden, womit die Öffnung am Umfang des Strahler-Detektor-Rings 10, wie in Figur 2 dargestellt, freigegeben wird. Eine Verkippung des Strahler-Detektor-Elements 26 gegenüber den restlichen Strahler-Detektor-Elementen 11, 12, 13, 14 tritt zu keinem Zeitpunkt auf. Durch das Fehlen von Kippmechanismen, was das Strahler-Detektor-Element 26 betrifft, kann der zur Verfügung stehende Bauraum, welcher sich über einen 72°-Winkel am Umfang des Strahler-Detektorrings 10 erstreckt, weitgehend für den Einbau röntgentechnischer Komponenten, auf welche im Folgenden noch näher eingegangen wird, genutzt werden.

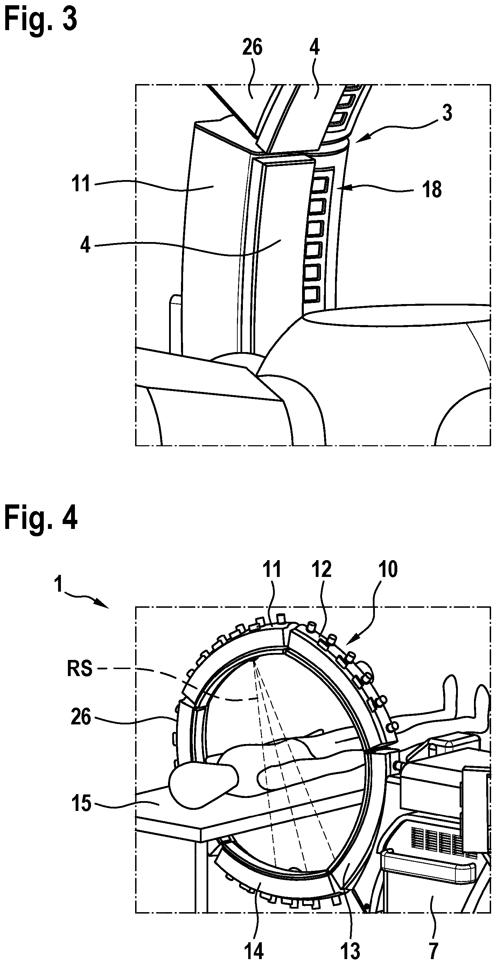

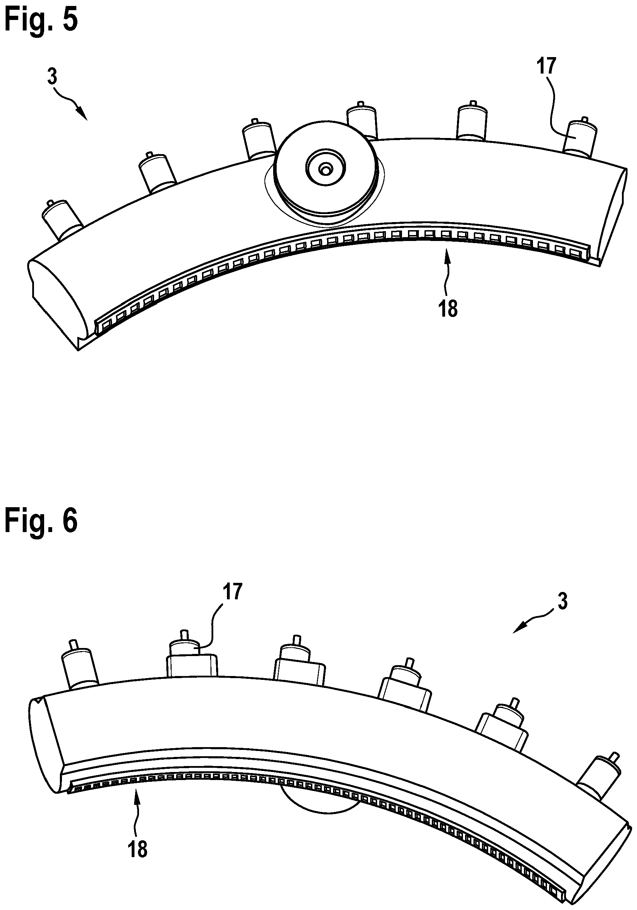

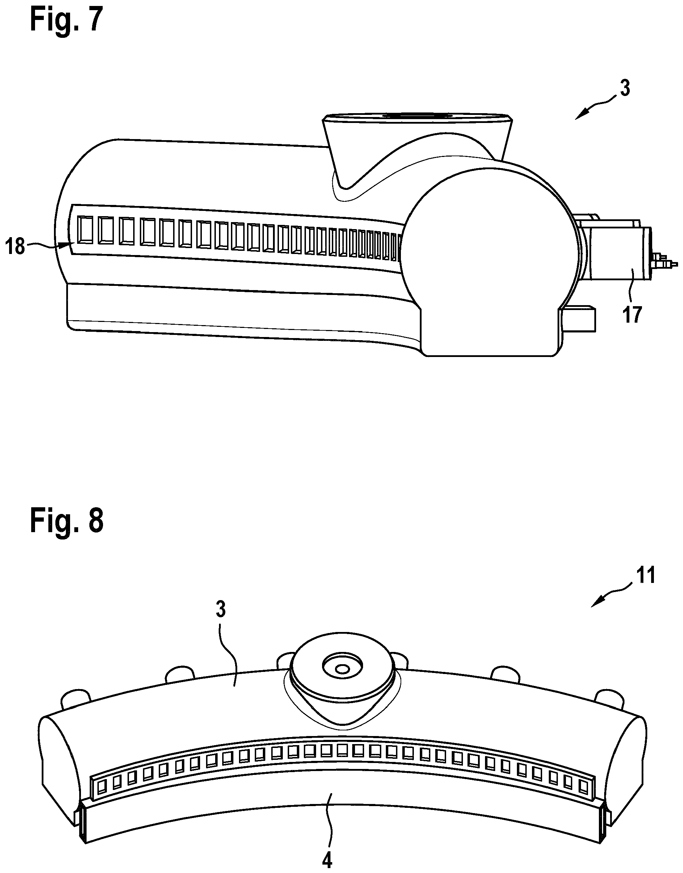

Hinsichtlich der Anordnung röntgentechnischer Komponenten unterscheidet sich das verschiebbare Strahler-Detektor-Element 26 nicht von den feststehenden Strahler-Detektor-Elementen 11, 12, 13, 14. Jedes Strahler-Detektor-Element 11, 12, 13, 14, 26 hat einen einheitlichen, in den Figuren 5 bis 8 dargestellten Aufbau. Anschlussstücke der Röntgenröhren 3 sind mit 17 bezeichnet. Strahler-Anordnungen 18 der Röntgenröhren 3 emittieren Röntgenstrahlung RS derart, dass stets, wie in Figur 4 veranschaulicht ist, zwei der fünf Strahler-Detektor-Elemente 11, 12, 13, 14, 26 - genauer: deren Detektoren 4 - getroffen werden. Durch die ungerade Anzahl der Strahler-Detektor-Elemente 11, 12, 13, 14, 26 liegt niemals eine Stoßstelle zwischen zwei Strahler-Detektor-Elementen 11, 12, 13, 14, 26 einer zweiten derartigen Stoßstelle genau diametral gegenüber.

In jeder Röntgenröhre 3 befindet sich eine Emitter-Baugruppe 19 zur Erzeugung von Elektronenstrahlen ES, die auf die Anodenanordnung 9 treffen und damit den Brennfleck BF erzeugen. Der Brennfleck BF hat nicht notwendigerweise eine annähernd punktförmige Gestalt. Vielmehr können in prinzipiell bekannter Weise zum Beispiel auch langgestreckte Brennflecke BF erzeugt werden, wobei die Lage des Brennflecks BF in jedem Fall als Lage dessen Mittelpunkts zu verstehen ist.

Im Ausführungsbeispiel umfasst die Emitter-Baugruppe 19 unterschiedliche Kathoden 5, 25, um Röntgenstrahlung unterschiedlicher Dosis und/oder Wellenlänge zu erzeugen. In jedem Fall werden Elektronen mit Hilfe eines Extraktionsgitters 20 aus der Kathode 5, 25 extrahiert, wobei der Elektronenstrahl ES mit Hilfe einer Elektrodenanordnung 21, die mehrere Elektroden 22, 23 umfasst, in definierter Weise auslenkbar ist. Eine Mehrzahl an Kathoden 5, 25 ist gemeinsam auf einer Platine 24 angeordnet.

Die gesamte mit der Emitter-Baugruppe 19 einer Röntgenröhre 3 zusammenwirkende Anodenanordnung 9 erstreckt sich am Umfang des Strahler-Detektor-Rings 10 über einen Winkel α, der signifikant weniger als 72° beträgt. Deutlich näher an 72° ist ein Winkel β, der die Erstreckung des Röntgendetektors 4 am Umfang des Strahler-Detektor-Rings angibt. Anders ausgedrückt: am Umfang des Strahler-Detektor-Rings 10 zwischen den einzelnen Röntgendetektoren 4 gebildete Lücken sind wesentlich enger als die zwischen den Strahler-Anordnungen 18 gebildeten Lücken. Eine Vielzahl möglicher Brennfleckpositionen erstreckt sich innerhalb der Röntgenröhre 3 über einen Winkel γ, der geringer als der Winkel α ist.

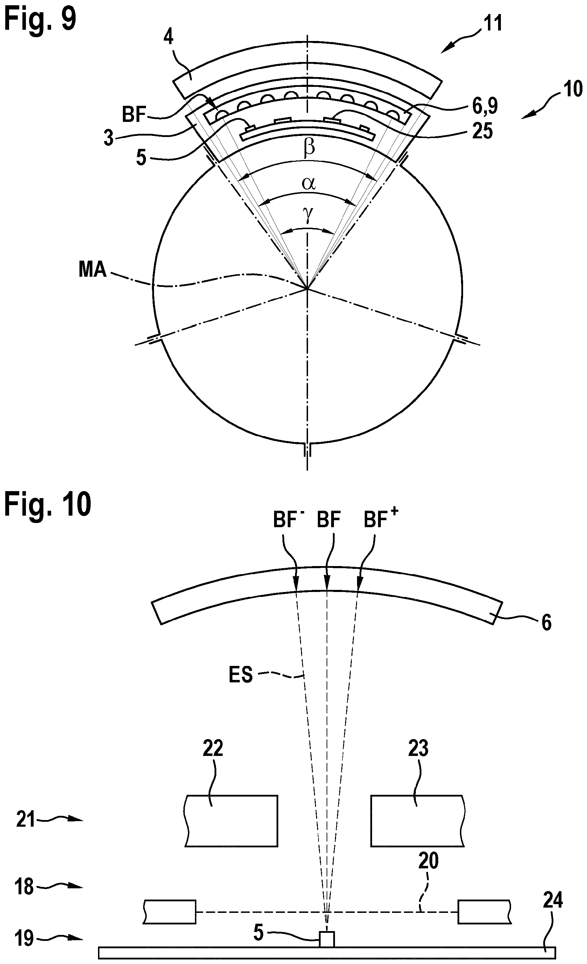

Die Elektrodenanordnung 21 ist dazu ausgebildet, den Elektronenstrahl ES wahlweise auf den Brennfleck BF oder einen im Vergleich hierzu in Umfangsrichtung des Strahler-Detektor-Rings 10 versetzten Brennfleck BF⁺, BF⁻ zu richten. Bezogen auf die Anordnung nach den Figuren 9 und 10 ist der Brennfleck BF⁺ gegenüber dem Brennfleck BF im Uhrzeigersinn und der Brennfleck BF⁻ im Gegenuhrzeigersinn ausgelenkt. Die Auslenkungen des Elektronenstrahls ES, welche einen Versatz des Brennflecks BF bedeuten, werden auch als Beam Toggling bezeichnet und ermöglichen eine besonders eng gestaffelte Platzierung von Brennflecken BF⁻, BF, BF⁺ am Umfang des Strahler-Detektor-Rings 10. Hierbei ist eine Gesamtzahl von mehreren hundert Brennfleckpositionen, was einem Mehrfachen der Anzahl der Elektronenemitter 5, 25 entspricht, erzielbar.

### Bezugszeichenliste

- 1: Computertomograph
- 2: Gantry
- 3: Röntgenröhre
- 4: Röntgendetektor
- 5: Kathode erster Art, Elektronenemitter
- 6: Anode
- 7: fahrbares Gestell
- 8: Bedien- und Auswerteeinheit
- 9: Anodenanordnung
- 10: Strahler-Detektor-Ring
- 11: erstes feststehendes Strahler-Detektor-Element
- 12: zweites feststehendes Strahler-Detektor-Element
- 13: drittes feststehendes Strahler-Detektor-Element
- 14: viertes feststehendes Strahler-Detektor-Element
- 15: Patientenliege
- 16: Schiebemechanismus
- 17: Anschlussstück
- 18: Strahler-Anordnung
- 19: Emitter-Baugruppe
- 20: Extraktionsgitter
- 21: Elektrodenanordnung
- 22: Elektrode
- 23: Elektrode
- 24: Platine
- 25: Kathode zweiter Art, Elektronenemitter
- 26: verschiebbares Strahler-Detektor-Element
- α: Winkel, über welchen sich die Anodenanordnung eines Strahler-Detektor Elements erstreckt
- β: Winkel, über welchen sich der Detektor eines Strahler-Detektor Elements erstreckt
- γ: Winkelbereich, in dem die möglichen Brennflecke einer Anodenanordnung liegen

- BF: Brennfleck (allgemein)
- BF⁺, BF⁻: Brennfleck, erzeugt mittels eines Elektronenemitters (in mittlerer Position sowie in zwei in Umfangsrichtung des Strahler-Detektor-Rings versetzten Positionen)
- ES: Elektronenstrahl
- MA: Mittelachse
- RS: Röntgenstrahlung

## Patentansprüche

1. Computertomograph, mit einem statischen Strahler-Detektor-Ring (10), welcher aus einer ungeraden Anzahl (n) an Strahler-Detektor-Elementen (11, 12, 13, 14, 26) aufgebaut ist, von welchen ein einziges (26) unter Öffnung des Strahler-Detektor-Rings (10) gegenüber den übrigen, zusammen eine C-Form beschreibenden Strahler-Detektor-Elementen (11, 12, 13, 14) verlagerbar ist, wobei jedes Strahler-Detektor-Element (11, 12, 13, 14, 26) eine zur Emission von Röntgenstrahlung vorgesehene Anodenanordnung (9), welche sich über einen Winkel α von mindestens 0,9 × 360°/n am Umfang des Strahler-Detektor-Rings (10) erstreckt, sowie einen zur Detektion von Röntgenstrahlung vorgesehenen Detektor (4), der sich innerhalb desselben Strahler-Detektor-Elements (11, 12, 13, 14, 26) über einen Winkel β von mindestens 0,95 × 360°/n erstreckt, aufweist, und wobei jede Anodenanordnung (9) Teil einer mehrere Elektronen-Emitter (5, 25) umfassenden Strahler-Anordnung (18) ist, in welcher jeder Elektronen-Emitter (5, 25) in Zusammenwirkung mit einer Elektrodenanordnung (21) zur Erzeugung eines Brennflecks (BF⁻, BF, BF⁺) an einer von mindestens drei wählbaren Positionen auf der Anodenanordnung (9) ausgebildet ist.

2. Computertomograph nach Anspruch 1, **dadurch gekennzeichnet, dass** das in Relation zum übrigen Strahler-Detektor-Ring (10) verlagerbare Strahler-Detektor-Element (26) in Axialrichtung des Strahler-Detektor-Rings (10) verschiebbar und im axial verschobenen Zustand in Tangentialrichtung an den insgesamt C-förmig angeordneten Strahler-Detektor-Elementen (11, 12, 13, 14) entlang schiebbar ist.

3. Computertomograph nach Anspruch 1 oder 2, **dadurch gekennzeichnet, dass** die Strahler-Detektor-Elemente (11, 12, 13, 14, 26) zur Feldemission von Elektronen ausgebildete Emitter (5, 25), insbesondere Kohlenstoffnanoröhren umfassende Emitter, aufweisen.

4. Computertomograph nach Anspruch 3, **dadurch gekennzeichnet, dass** jedes Strahler-Detektor-Element (11, 12, 13, 14, 26) mindestens einen Emitter (5) ersten Typs sowie mindestens einen Emitter (25) zweiten Typs aufweist.

5. Computertomograph nach Anspruch 4, **dadurch gekennzeichnet, dass** sich die unterschiedlichen Emitter-Typen (5, 25) innerhalb eines Strahler-Detektor-Elements (11, 12, 13, 14, 26) hinsichtlich ihrer Materialien und/oder Geometrie voneinander unterscheiden.

6. Computertomograph nach einem der Ansprüche 3 bis 5, **dadurch gekennzeichnet, dass** die Strahler-Detektor-Elemente (11, 12, 13, 14, 26) zur Umschaltung zwischen verschiedenen Röntgenfrequenzen und/oder Röntgendosen ausgebildet sind, wobei jeder Brennfleck (BF⁻, BF, BF⁺) gleichermaßen als Quelle sämtlicher einstellbarer Röntgenfrequenzen und Röntgendosen wählbar ist.

7. Computertomograph nach einem der Ansprüche 1 bis 6, **dadurch gekennzeichnet, dass** der Strahler-Detektor-Ring (10) an einem fahrbaren Gerätegestell (7) in verstellbarer Weise angebracht ist.

8. Computertomograph nach einem der Ansprüche 1 bis 7, **dadurch gekennzeichnet, dass** der Strahler-Detektor-Ring (10) mindestens fünf und höchstens neun Strahler-Detektor-Elemente (11, 12, 13, 14, 26) umfasst, wobei sämtliche Strahler-Detektor-Elemente (11, 12, 13, 14, 26) einschließlich des verlagerbaren Strahler-Detektor-Elements (26) gleich große Winkelbereiche abdecken.

9. Computertomograph nach einem der Ansprüche 1 bis 8, **dadurch gekennzeichnet, dass** zwischen den am weitesten voneinander entfernten Brennflecken (BF⁻, BF⁺) derselben Anodenanordnung (9) ein Winkel γ von mindestens 0,85 × α am Umfang des Strahler-Detektor-Rings eingeschlossen ist und von jeder der möglichen Brennfleckpositionen (BF⁻, BF, BF⁺) aus ein fächerförmiges Röntgenstrahlenbündel auf mindestens zwei dem Brennfleck diametral am Strahler-Detektor-Ring (10) gegenüberliegende Strahler-Detektor-Elemente (11, 12, 13, 14, 26) ausrichtbar ist.

10. Computertomograph nach einem der Ansprüche 1 bis 9, **dadurch gekennzeichnet, dass** die Strahler-Detektor-Elemente (11, 12, 13, 14, 26) zur gleichzeitigen Erzeugung mindestens zweier am Umfang des Strahler-Detektor-Rings (10) gegeneinander versetzter Brennflecke (BF⁻, BF, BF⁺) ausgebildet sind.

11. Verfahren zum Betrieb eines Computertomographen (1), welcher einen nicht rotierenden Strahler-Detektor-Ring (10) umfasst, der aus einer ungeraden Anzahl an Strahler-Detektor-Elementen (11, 12, 13, 14, 26) aufgebaut ist, von welchen ein einziges Element (26) zum Öffnen des Strahler-Detektor-Rings (10) ausgebildet ist, wobei sowohl in den festen Strahler-Detektor-Elementen (11, 12, 13, 14) als auch im zu öffnenden Strahler-Detektor-Element (26) eine Mehrzahl an Elektronen-Emittern (5, 25) angeordnet ist, die jeweils dazu ausgebildet sind, mit Hilfe von Elektronenstrahlen beeinflussenden Elektroden (21, 22, 23) einen Brennfleck (BF⁻, BF, BF⁺) mit variabler Position auf einer dem Strahler-Detektor-Element (11, 12, 13, 14, 26) zugehörigen Anode (6) zu erzeugen, so dass die Gesamtzahl möglicher Brennfleckpositionen einem Mehrfachen der Anzahl an Elektronen-Emittern (5, 25) entspricht, und wobei der maximale Winkelabstand zwischen zwei in Umfangsrichtung des Strahler-Detektor-Rings (10) innerhalb ein und desselben Strahler-Detektor-Elementes (11, 12, 13, 14, 26) nebeneinander angeordneten Brennfleckpositionen geringer ist als der minimale Winkelabstand zwischen Brennfleckpositionen zweier benachbarter Strahler-Detektor-Elemente (11, 12, 13, 14, 26), mit folgenden Schritten:
- Positionierung des Strahler-Detektor-Rings (10) um ein Untersuchungsobjekt, wobei der Strahler-Detektor-Ring (10) spätestens in einer zur Durchführung einer röntgentechnischen Untersuchung vorgesehenen Position geschlossen wird,
- Richten eines fächerförmigen Röntgenstrahlenbündels, welches von einem ersten Brennfleck (BF⁻, BF, BF⁺) ausgeht, auf das Untersuchungsobjekt, wobei Röntgenstrahlung von Detektoren (4) mindestens zweier Strahler-Detektor-Elemente (11, 12, 13, 14, 26) detektiert wird,
- Erzeugen eines zweiten Brennflecks (BF⁻, BF, BF⁺), welcher am Umfang des Strahler-Detektor-Rings (10) gegenüber dem ersten Brennfleck (BF⁻, BF, BF⁺) um einen ersten Differenzwinkel versetzt ist,
- Erzeugen weiterer Brennflecke (BF⁻, BF, BF⁺), welche am Umfang des Strahler-Detektor-Rings (10) jeweils gegenüber dem vorherigen Brennfleck (BF⁻, BF, BF⁺) um einen Differenzwinkel versetzt sind, wobei der Differenzbetrag zwischen zwei aufeinanderfolgenden Differenzwinkeln geringer ist als die Differenz zwischen dem minimalen Winkelabstand zwischen Brennfleckpositionen in benachbarten Strahler-Detektor-Elementen (11, 12, 13, 14, 26) und dem maximalen Winkelabstand zwischen zwei benachbarten Brennfleckpositionen innerhalb ein und desselben Strahler-Detektor-Elementes (11, 12, 13, 14, 26).

12. Verfahren nach Anspruch 11, **dadurch gekennzeichnet, dass** durch die sukzessive Umschaltung zwischen verschiedenen Brennfleckpositionen mehrere Umläufe um die Mittelachse (MA) des Strahler-Detektor-Rings (10) beschrieben werden, wobei erst nach einer Mehrzahl an Umläufen sämtliche mögliche Brennfleckpositionen eingenommen wurden.

13. Verfahren nach Anspruch 12, **dadurch gekennzeichnet, dass** bei jedem einzelnen Umlauf Brennflecke (BF⁻, BF, BF⁺) in voneinander abweichenden Einstellungen verschiedener Elektronenstrahlen beeinflussenden Elektroden (21, 22, 23) erzeugt werden.

## Claims

1. A computer tomograph comprising a static radiator-detector ring (10) which is constructed from an odd number (n) of radiator-detector elements (11, 12, 13, 14, 26), a single radiator-detector element (26) thereof being displaceable, by opening the radiator-detector ring (10), in relation to the remaining radiator-detector elements (11, 12, 13, 14) which together describe a C-shape, wherein each radiator-detector element (11, 12, 13, 14, 26) has an anode arrangement (9) which is provided for the emission of X-rays and which extends over an angle α of at least 0.9 × 360°/n on the circumference of the radiator-detector ring (10), as well as a detector (4) which is provided for detecting X-rays and which extends within the same radiator-detector element (11, 12, 13, 14, 26) thereof over an angle β of at least 0.95 × 360°/n, and wherein each anode arrangement (9) is part of a radiator arrangement (18) which comprises a plurality of electron emitters (5, 25) and in which each electron emitter (5, 25) is configured in cooperation with an electrode arrangement (21) to generate a focal spot (BF-, BF, BF+) at one of at least three selectable positions on the anode arrangement (9).

2. The computer tomograph according to Claim 1, **characterized in that** the radiator-detector element (26) which is displaceable in relation to the remaining radiator-detector ring (10) can be displaced in the axial direction of the radiator-detector ring (10), and in the axially displaced state is slidable in the tangential direction along the radiator-detector elements (11, 12, 13, 14) which are arranged as a whole in a C-shape.

3. The computer tomograph according to Claim 1 or 2, **characterized in that** the radiator-detector elements (11, 12, 13, 14, 26) have emitters (5, 25) which are configured for the field emission of electrons, in particular emitters comprising carbon nanotubes.

4. The computer tomograph according to Claim 3, **characterized in that** each radiator-detector element (11, 12, 13, 14, 26) has at least one emitter (5) of the first type and at least one emitter (25) of the second type.

5. The computer tomograph according to Claim 4, **characterized in that** the different emitter types (5, 25) within a radiator-detector element (11, 12, 13, 14, 26) differ from one another regarding their materials and/or geometry.

6. The computer tomograph according to one of Claims 3 to 5, **characterized in that** the radiator-detector elements (11, 12, 13, 14, 26) are configured to switch between different X-ray frequencies and/or X-ray doses, wherein each focal spot (BF-, BF, BF+) is equally selectable as the source of all settable X-ray frequencies and X-ray doses.

7. The computer tomograph according to one of Claims 1 to 6, **characterized in that** the radiator-detector ring (10) is attached in an adjustable manner to a movable device frame (7).

8. The computer tomograph according to one of Claims 1 to 7, **characterized in that** the radiator-detector ring (10) comprises at least five and at most nine radiator-detector elements (11, 12, 13, 14, 26), wherein all radiator-detector elements (11, 12, 13, 14, 26) including the displaceable radiator-detector element (26) cover angular ranges of the same size.

9. The computer tomograph according to one of Claims 1 to 8, **characterized in that** an angle γ of at least 0.85 × α on the circumference of the radiator-detector ring is enclosed between the focal spots (BF-, BF+) furthest distant from one another of the same anode arrangement (9), and from each of the possible focal spot positions (BF⁻, BF, BF⁺) a fan-shaped X-ray beam can be aligned with at least two radiator-detector elements (11, 12, 13, 14, 26) diametrically opposing the focal spot on the radiator-detector ring (10).

10. The computer tomograph according to one of Claims 1 to 9, **characterized in that** the radiator-detector elements (11, 12, 13, 14, 26) are configured to generate at the same time at least two mutually offset focal spots (BF⁻, BF, BF⁺) on the circumference of the radiator-detector ring (10).

11. A method for operating a computer tomograph (1) which comprises a non-rotating radiator-detector ring (10) which is constructed from an odd number of radiator-detector elements (11, 12, 13, 14, 26), a single element (26) thereof being configured to open the radiator-detector ring (10), wherein a plurality of electron emitters (5, 25) is arranged both in the fixed radiator-detector elements (11, 12, 13, 14) and in the radiator-detector element (26) to be opened, which electron emitters are configured to generate, in each case by means of electrodes (21, 22, 23) influencing electron beams, a focal spot (BF⁻, BF, BF⁺) having a variable position on an anode (6) associated with the radiator-detector element (11, 12, 13, 14, 26) so that the total number of possible focal spot positions corresponds to a multiple of the number of electron emitters (5, 25), and wherein the maximum angular spacing between two focal spot positions arranged adjacent to one another within one and the same radiator-detector element (11, 12, 13, 14, 26) in the circumferential direction of the radiator-detector ring (10) is less than the minimum angular spacing between the focal spot positions of two adjacent radiator-detector elements (11, 12, 13, 14, 26), comprising the following steps:
- positioning the radiator-detector ring (10) around an object to be examined, wherein the radiator-detector ring (10) is closed at the latest in a position provided for carrying out an X-ray examination,
- directing a fan-shaped X-ray beam which originates from a first focal spot (BF⁻, BF, BF⁺) onto the object to be examined, wherein X-rays are detected by detectors (4) of at least two radiator-detector elements (11, 12, 13, 14, 26),
- generating a second focal spot (BF⁻, BF, BF⁺) which is offset by a first differential angle relative to the first focal spot (BF⁻, BF, BF⁺) on the circumference of the radiator-detector ring (10),
- generating further focal spots (BF⁻, BF, BF⁺) which are offset in each case by a differential angle relative to the previous focal spot (BF⁻, BF, BF⁺) on the circumference of the radiator-detector ring (10), wherein the difference between two successive differential angles is less than the difference between the minimum angular spacing between the focal spot positions in adjacent radiator-detector elements (11, 12, 13, 14, 26) and the maximum angular spacing between two adjacent focal spot positions within one and the same radiator-detector element (11, 12, 13, 14, 26).

12. The method according to Claim 11, **characterized in that** a plurality of revolutions about the central axis (MA) of the radiator-detector ring (10) are described by successively switching between the different focal spot positions, wherein all possible focal spot positions have been assumed only after a plurality of revolutions.

13. The method according to Claim 12, **characterized in that** with each individual revolution focal spots (BF⁻, BF, BF⁺) are generated in mutually different settings of various electrodes (21, 22, 23) influencing electron beams.

## Revendications

1. Tomographe informatique, doté d'un anneau de détection de rayonnement statique (10), qui est construit à partir d'un nombre impair (n) d'éléments détecteurs de rayonnements (11, 12, 13, 14, 26), dont un seul (26) peut être déplacé en ouvrant l'anneau de détection de rayonnement (10) par rapport aux éléments détecteurs de rayonnement restants (11, 12, 13, 14), qui forment conjointement une forme de C, dans lequel chaque élément détecteur de rayonnement (11, 12, 13, 14, 26) présente un agencement d'anodes (9) prévu pour l'émission de rayons X, qui s'étend sur un angle α d'au moins 0,9×360°/n sur la circonférence de l'anneau de détection de rayonnement (10), ainsi qu'un détecteur (4) prévu pour la détection de rayons X , qui s'étend sur un angle β d'au moins 0,95x360°/n à l'intérieur de cet élément détecteur de rayonnement (11, 12, 13, 14, 26) et dans lequel chaque agencement d'anodes (9) fait partie d'un agencement de rayonnements (18) comprenant plusieurs émetteurs d'électrons (5, 25), dans lequel chaque émetteur d'électrons (5, 25) est conçu en coopération avec un agencement d'électrodes (21) pour générer un point focal (BF⁻, BF, BF⁺) au niveau d'une d'au moins trois positions sélectionnables sur l'agencement d'anodes (9) .

2. Tomographe informatique selon la revendication 1, **caractérisé en ce que** l'élément détecteur de rayonnement (26), déplaçable par rapport à l'anneau de détection de rayonnement restant (10), peut être déplacé dans la direction axiale de l'anneau de détection de rayonnement (10) et à l'état déplacé axialement dans la direction tangentielle, peut être poussé le long des éléments détecteurs de rayonnements (11, 12, 13, 14) qui sont disposés en forme de C.

3. Tomographe informatique selon la revendication 1 ou 2, **caractérisé en ce que** les éléments détecteurs de rayonnement (11, 12, 13, 14, 26) présentent des émetteurs (5, 25), notamment des émetteurs comprenant des nanotubes de carbone, conçus pour l'émission de champ des électrons.

4. Tomographe informatique selon la revendication 3, **caractérisé en ce que** chaque élément détecteur de rayonnement (11, 12, 13, 14, 26) présente au moins un émetteur (5) du premier type et au moins un émetteur (25) du deuxième type.

5. Tomographe informatique selon la revendication 4, **caractérisé en ce que** les différents types d'émetteurs (5, 25) diffèrent les uns des autres en termes de matériaux et/ou de géométrie à l'intérieur d'un élément de détecteur de rayonnement (11, 12, 13, 14, 26) .

6. Tomographe informatique selon une des revendications 3 à 5, **caractérisé en ce que** les éléments détecteurs de rayonnements (11, 12, 13, 14, 26) sont conçus pour commuter entre différentes fréquences de rayons X et/ou doses de rayons X, dans lequel chaque point focal (BF⁻, BF, BF⁺) peut être sélectionné aussi bien comme source de toutes les fréquences de rayons X réglables et doses de rayons X.

7. Tomographe informatique selon une des revendications 1 à 6, **caractérisé en ce que** l'anneau de détection de rayonnement (10) est fixé de manière réglable sur un cadre d'appareil mobile (7).

8. Tomographe informatique selon une des revendications 1 à 7, **caractérisé en ce que** l'anneau de détection de rayonnements (10) comprend au moins cinq et au plus neuf éléments détecteurs de rayonnements (11, 12, 13, 14, 26), dans lequel tous les éléments détecteurs de rayonnement (11, 12, 13, 14, 26), y compris l'élément détecteur de rayonnement mobile (26), couvrent des plages angulaires de même taille.

9. Tomographe informatique selon une des revendications 1 à 8, **caractérisé en ce qu'**entre les points focaux les plus éloignés (BF⁻, BF⁺) du même agencement d'anodes (9) est défini un angle y d'au moins 0,85 × α sur la circonférence de l'anneau de détection de rayonnement et un faisceau de rayons X en forme d'éventail provenant de chacune des positions de points focaux possibles (BF⁻, BF, BF⁺) peut être aligné sur au moins deux éléments détecteurs de rayonnement (11, 12, 13, 14, 26) situés diamétralement opposés au point focal sur l'anneau de détection de rayonnement (10).

10. Tomographe informatique selon une des revendications 1 à 9, **caractérisé en ce que** les éléments détecteurs de rayonnements (11, 12, 13, 14, 26) sont utilisés pour générer simultanément au moins deux points focaux décalés l'un de l'autre sur la circonférence de l'anneau de détecteur de rayonnement (BF⁻, BF, BF⁺).

11. Procédé de fonctionnement d'un tomographe informatique (1), qui comprend un anneau de détection de rayonnement non rotatif (10) qui est construit à partir d'un nombre impair d'éléments détecteurs de rayonnements (11, 12, 13, 14, 26), dont un seul élément (26) est conçu pour ouvrir l'anneau de détection de rayonnement (10), dans lequel tant dans les éléments détecteurs de rayonnement fixes (11, 12, 13, 14) que dans l'élément détecteur de rayonnement à ouvrir (26) une pluralité d'émetteurs d'électrons (5, 25) sont disposés, qui sont respectivement conçus pour créer un point focal (BF⁻, BF, BF⁺) à position variable à l'aide d'électrodes (21, 22, 23) influençant des faisceaux d'électrons sur une anode (6) associée à l'élément détecteur de rayonnement (11, 12, 13, 14, 26), de sorte que le nombre total de positions de point focal possibles soit un multiple du nombre d'émetteurs d'électrons (5, 25), et dans lequel la distance angulaire maximale entre deux positions de point focal disposées l'une à côté de l'autre dans la direction circonférentielle de l'anneau de détection de rayonnement (10) à l'intérieur d'un même élément détecteur de rayonnement (11, 12, 13, 14, 26) est inférieure à la distance angulaire minimale entre les positions des points focaux de deux éléments détecteurs de rayonnements adjacents (11, 12, 13, 14, 26), comportant les étapes suivantes consistant à :
- positionner l'anneau de détection de rayonnement (10) autour d'un objet à examiner, dans lequel l'anneau de détection de rayonnement (10) est fermé au plus tard dans une position destinée à réaliser un examen radiologique,
- diriger un faisceau de rayons X en forme d'éventail, qui émane d'un premier point focal (BF⁻, BF, BF⁺) , sur l'objet à examiner, dans lequel les rayons X sont détectés par des détecteurs (4) d'au moins deux éléments détecteurs de rayonnement (11, 12, 13, 14, 26),
- générer un deuxième point focal (BF⁻, BF, BF⁺) , qui est décalé d'un premier angle différent sur la circonférence de l'anneau de détection de rayonnement (10) par rapport au premier point focal (BF⁻; BF, BF⁺) ,
- générer d'autres points focaux (BF⁻, BF, BF⁺) , qui sont chacun décalés d'un angle différent sur la circonférence de l'anneau de détection de rayonnement (10) par rapport au point focal précédent (BF⁻, BF, BF⁺) , dans lequel la proportion entre deux angles de différence consécutifs est ainsi inférieure à la différence entre la distance angulaire minimale entre des positions de points focaux dans les éléments détecteurs émetteurs adjacents (11, 12, 13, 14, 26) et la distance angulaire maximale entre deux positions de points focaux adjacentes à l'intérieur d'un seul et même élément détecteur de rayonnement (11, 12, 13, 14, 26).

12. Procédé selon la revendication 11, **caractérisé en ce que** par commutation successive entre différentes positions de point focal, on décrit plusieurs révolutions autour de l'axe central (MA) de l'anneau de détection de rayonnement (10), dans lequel toutes les positions de point focal possibles n'interviennent qu'après une pluralité de révolutions.

13. Procédé selon la revendication 12, **caractérisé en ce que** pour chaque révolution individuelle, des points focaux (BF⁻, BF, BF⁺) sont générés dans différents réglages de différentes électrodes (21, 22, 23) influençant les faisceaux d'électrons.
